(19) Europäisches Patentamt
European Patent Office
Office européen des brevets

(11) Veröffentlichungsnummer: **0 042 513**
**B1**

# (12) EUROPÄISCHE PATENTSCHRIFT

(45) Veröffentlichungstag der Patentschrift: **08.08.84**

(51) Int. Cl.³: **C 07 D 237/04**, A 61 K 31/50

(21) Anmeldenummer: **81104262.1**

(22) Anmeldetag: **03.06.81**

(54) **Neue Dihydropyridazinone, Verfahren zu ihrer Herstellung und diese Verbindungen enthaltende therapeutische Mittel.**

(30) Priorität: **13.06.80 DE 3022177**
**08.09.80 DE 3033702**

(43) Veröffentlichungstag der Anmeldung:
**30.12.81 Patentblatt 81/52**

(45) Bekanntmachung des Hinweises auf die Patenterteilung:
**08.08.84 Patentblatt 84/32**

(84) Benannte Vertragsstaaten:
**AT BE CH DE FR GB IT LI LU NL SE**

(56) Entgegenhaltungen:
**DE - A - 2 157 453**
**US - A - 3 689 652**
**US - A - 4 011 321**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Thyes, Marco, Dr., Thorwaldsenstrasse 1,
D-6700 Ludwigshafen (DE)**
Erfinder: **Franke, Albrecht, Dr., Mandelring 11,
D-6706 Wachenheim (DE)**
Erfinder: **Koenig, Horst, Dr., Pariser Strasse 4,
D-6700 Ludwigshafen (DE)**
Erfinder: **Lenke, Dieter, Dr., Kekuleplatz 1,
D-6700 Ludwigshafen (DE)**
Erfinder: **Lehmann, Hans Dieter, Dr., Im Hefen 15,
D-6945 Hirschberg-Leutershausen (DE)**
Erfinder: **Gries, Josef, Dr., Romerweg 43,
D-6706 Wachenheim (DE)**

**EP 0 042 513 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 6-Phenyl-4,5-dihydro-3(2 H)-pyridazinone, die im Phenylring in p-Stellung durch eine Carbamat- oder Thiocarbamatgruppe substituiert sind, Verfahren zu ihrer Herstellung, diese Verbindungen enthaltende pharmazeutische Zubereitungen und ihre Verwendung als Arzneimittel bei thrombo-embolischen Erkrankungen und als Antihypertensiva.

6-(Acylamino)-phenyl-4,5-dihydro-3(2 H)-pyridazinone sind bereits mehrfach beschrieben worden, beispielsweise werden in der DE-OS 1 670 158 in den Stellungen 4 und 5 unsubstituierte 6-(Acylamino)phenyl-4,5-dihydro-3(2 H)-pyridazinone mit blutdrucksenkenden und entzündungshemmenden Eigenschaften beschrieben. Für 6-Phenyl-4,5-dihydro-3(2 H)-pyridazinone, die in 4-Stellung eine Alkylgruppe tragen und im Phenylrest in p-Stellung durch eine Gruppe der Formel $-NHR^4$, in der $R^4$ beispielsweise für einen Acylrest oder einen Ethoxycarbonylrest steht, substituiert sind, werden in der DE-OS 2 304 977 cardiovasculäre und antiphlogistische Eigenschaften genannt. In der DE-OS 2 150 436 und den US-PS 3 824 271 und 3 888 901 werden blutdrucksenkend wirkende, in 5-Stellung durch einen Alkylrest substituierte 6-(Alkanoylamino)phenyl-4,5-dihydro-3(2 H)-pyridazinone beschrieben. Weiterhin gehen aus der DE-OS 2 727 481 und der deutschen Patentanmeldung P 2 854 191.5 6-(p-Alkanoylaminophenyl)-4,5-dihydro-3(2 H)-pyridazinone, die in der Alkanoylgruppe durch ein oder mehrere Halogenatome substituiert sind, als Arzneimittel wegen ihrer thrombozytenaggregationshemmenden und blutdrucksenkenden Eigenschaften hervor.

In der DE-OS 2 123 246 werden blutdrucksenkend, coronarerweiternd und antiinflammatorisch wirkende 6-(p-Alkanoylaminophenyl)-4,5-dihydro-3(2 H)-pyridazinone, die im Alkanoylrest eine substituierte Aminogruppe tragen, beschrieben. Für 6-Phenyl-4,5-dihydro-3(2 H)-pyridazinone, die im Phenylrest in p-Stellung durch eine Gruppe der Formel $-NHCONR^5R^6$, in der die Reste $R^5$ und $R^6$ gleich oder verschieden sind und beispielsweise für Wasserstoff, eine Alkylgruppe oder eine Arylgruppe stehen, substituiert sind, werden in der DE-OS 2 157 453 cardiovasculäre und antiinflammatorische Eigenschaften angegeben. Schließlich werden in der japanischen Patentanmeldung 53 124-279 antiallergisch, membranstabilisierend und thrombozytenaggregationshemmend wirkende 6-[p-(Alkoxycarbonylaminoalkyl)phenyl]-4,5-dihydro-3(2 H)-pyridazinone beschrieben.

Es wurde nun gefunden, daß 6-Aryl-4,5-dihydro-3(2 H)-pyridazinone der allgemeinen Formel I,

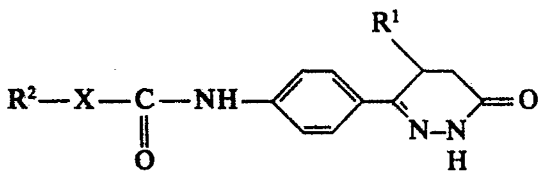

(I)

in der

X ein Sauerstoffatom oder ein Schwefelatom,

$R^1$ einen Alkylrest mit 1 bis 3 C-Atomen und

$R^2$ einen Alkylrest mit 1 bis 8 C-Atomen, der gegebenenfalls durch ein bis vier Halogenatome, eine Alkoxygruppe mit 1 bis 4 C-Atomen im Alkyl, die mindestens durch zwei C-Atome von X getrennt ist, eine Cycloalkylgruppe mit 3 bis 8 C-Atomen im Ring, die gegebenenfalls ein oder zwei Alkylgruppen mit 1 bis 3 C-Atomen aufweist, oder einen Phenylrest, der gegebenenfalls ein bis drei gleiche oder verschiedene Substituenten aus der Gruppe Alkyl mit 1 bis 3 C-Atomen, Alkoxy mit 1 bis 3 C-Atomen im Alkyl, Halogen, Trifluormethyl oder Nitro aufweist, substituiert ist, einen Cycloalkylrest mit 3 bis 8 C-Atomen im Ring, der gegebenenfalls ein- bis vierfach durch Alkylreste mit 1 bis 4 C-Atomen substituiert ist, einen Alkenylrest mit 3 bis 8 C-Atomen, einen Alkinylrest mit 3 bis 8 C-Atomen oder einen Phenylrest, der gegebenenfalls durch ein bis drei gleiche oder verschiedene Substituenten aus der Gruppe Alkyl mit 1 bis 4 C-Atomen, Alkoxy mit 1 bis 4 C-Atomen im Alkyl, Halogen, Trifluormethyl, Cyan oder Nitro substituiert ist,

bedeuten, wertvolle pharmakologische Eigenschaften aufweisen.

Alkylreste mit 1 bis 3 C-Atomen für $R^1$ sind insbesondere Methyl, Ethyl und Propyl.

Als unsubstituierte Alkylreste mit 1 bis 8 C-Atomen für den Rest $R^2$, geradkettig oder verzweigt, seien Methyl, Ethyl, Propyl, Isopropyl, Butyl, Isobutyl, sec-Butyl, tert.-Butyl, Pentyl, 2-Methylbutyl und Isopentyl genannt.

Die bevorzugten unsubstituierten Alkylreste für $R^2$ sind solche, die ein bis vier C-Atome enthalten.

Durch ein bis vier Halogenatome, wie Chlor, Brom oder Fluor, substituierte Alkylreste mit 1 bis 8 C-Atomen für den Rest $R^2$, geradkettig oder verzweigt, sind beispielsweise

Chlormethyl, Brommethyl, Dichlormethyl, Trifluormethyl, 1-Chlorethyl, 2-Chlorethyl, 2-Bromethyl, 2-Fluorethyl, 1,2-Dichlorethyl, 1,1,2-Trichlorethyl, 2,2,2-Trichlorethyl, 2,2,2-Tribromethyl, 2,2,2-Trifluorethyl, 2-Brompropyl, 3-Chlorpropyl, 2,3-Dichlorpropyl, 2,3-Dibrompropyl, 3-Brom-2-chlorpropyl, 2-Chlorisopropyl, 2,2'-Dichlorisopropyl, 2-Chlorbutyl, 4-Chlorbutyl, 1-Chlormethylpropyl, 3,4-Dibrombutyl, 2-Chlorisobutyl und 3-Chlorisobutyl.

Von den Halogenalkylresten für $R^2$ sind solche bevorzugt, die 1 bis 4 C-Atome und ein bis drei

Halogenatome, insbesondere Chlor, Brom oder Fluor, enthalten.

Alkoxyalkylreste für $R^2$, die gebildet sind aus der Kombination einer Alkoxygruppe mit 1 bis 4 C-Atomen im Alkyl und einer geradkettigen oder verzweigten Alkylengruppe mit 2 bis 8 C-Atomen und in denen die Alkoxygruppe so angeordnet ist, daß sie mindestens durch eine Zweikohlenstoffkette von X getrennt ist, sind zum Beispiel

2-Methoxyethyl, 2-Ethoxyethyl, 2-Propoxyethyl, 2-Isopropoxyethyl, 2-Butoxyethyl, 2-Methoxypropyl, 2-Ethoxypropyl, 3-Methoxypropyl, 3-Ethoxypropyl, 2-Methoxyisopropyl, 2-Ethoxyisopropyl, 2-Methoxybutyl, 3-Methoxybutyl, 4-Methoxybutyl und 1,1-Dimethyl-2-methoxyethyl.

Vorzugsweise sind die Alkoxyalkylreste für $R^2$ aus der Kombination einer Alkoxygruppe mit 1 bis 3 C-Atomen im Alkyl und einer Alkylengruppe mit 2 bis 4 C-Atomen gebildet.

Für $R^2$ kommen als geradkettige oder verzweigte Alkylreste mit 1 bis 8 C-Atomen, die durch eine gegebenenfalls bis zu zwei Alkylgruppen mit 1 bis 3 C-Atomen tragende Cycloalkylgruppe mit 3 bis 8 C-Atomen im Ring substituiert sind, beispielsweise

Cyclopropylmethyl, (1-Methylcyclopropyl)methyl, (2-Methylcyclopropyl)methyl, (2,3-Dimethylcyclopropyl)methyl, Cyclobutylmethyl, (3-Methylcyclobutyl)-methyl, Cyclopentylmethyl, Cyclohexylmethyl, 1-Cyclopropylethyl, 1-Cyclobutylethyl, 1-Cyclopentylethyl, 2-Cyclopropylethyl, 2-Cyclobutylethyl, 2-Cyclopentylethyl, 1-Cyclopropylpropyl, 2-Cyclobutylpropyl, 3-Cyclopropylpropyl, 3-Cyclopentylpropyl, 1-Cyclopropyl-1-methylethyl, 2-Cyclohexyl-1-methylethyl und 4-Cyclohexylbutyl in Betracht.

Von den Cycloalkylalkylresten für $R^2$ sind solche bevorzugt, die gebildet sind aus der Kombination einer gegebenenfalls substituierten Cycloalkylgruppe mit 3 bis 6 C-Atomen im Ring und einer Alkylengruppe mit 1 bis 3 C-Atomen.

Durch eine Phenylgruppe substituierte Alkylreste mit 1 bis 8 C-Atomen für den Rest $R^2$, geradkettig oder verzweigt, sind zum Beispiel

Benzyl, 1-Phenylethyl, 2-Phenylethyl, 1-Phenylpropyl, 2-Phenylpropyl, 3-Phenylpropyl, 1-Methyl-2-phenylethyl, 1-Methyl-1-phenylethyl und 4-Phenylbutyl.

Die bevorzugten Phenylalkylreste für $R^2$ sind solche, die gebildet sind aus der Kombination einer Phenylgruppe und einer Alkylengruppe mit 1 bis 4 C-Atomen.

Arylalkylreste für $R^2$, die gebildet sind aus der Kombination einer ein bis drei gleiche oder verschiedene Substituenten aus der Gruppe Alkyl mit 1 bis 3 C-Atomen, wie Methyl, Ethyl oder Propyl, Alkoxy mit 1 bis 3 C-Atomen im Alkyl, wie Methoxy oder Ethoxy, Halogen, wie Chlor, Brom oder Fluor, Trifluormethyl oder Nitro tragenden Phenylgruppe und einer geradkettigen oder verzweigten Alkylengruppe mit 1 bis 8 C-Atomen sind beispielsweise

o-Methyl-benzyl, m-Methylbenzyl, p-Methylbenzyl, p-Ethylbenzyl, o-Methoxybenzyl, m-Methoxybenzyl, p-Methoxybenzyl, o-Ethoxybenzyl, m,p-Dimethoxybenzyl, m,m',p-Trimethoxybenzyl, o-Chlorbenzyl, m-Chlorbenzyl, p-Chlorbenzyl, o-Fluorbenzyl, m-Fluorbenzyl, p-Fluorbenzyl, m-Trifluormethylbenzyl, o-Nitrobenzyl, p-Nitrobenzyl, 1-(m-Methoxy-phenyl)ethyl, 2-(m,p-Dimethoxyphenyl)ethyl, 2-(p-Fluorphenyl)ethyl und 2-(o-Nitrophenyl)ethyl.

Vorzugsweise sind die Arylalkylreste für $R^2$ aus der Kombination einer substituierten Phenylgruppe und einer Alkylengruppe mit 1 bis 4 C-Atomen gebildet.

Für den Rest $R^2$ kommen als gegebenenfalls ein- bis vierfach durch Alkylreste mit 1 bis 4 C-Atomen, wie Methyl, Ethyl oder Propyl, substituierte Cycloalkylgruppen mit 3 bis 8 C-Atomen im Ring zum Beispiel

Cyclopropyl, 1-Methyl-cyclopropyl, 2-Methylcyclopropyl, 1-Ethylcyclopropyl, 2,2-Dimethylcyclopropyl, 1,2, 2-Trimethylcyclopropyl, Cyclobutyl, 1-Methylcyclobutyl, 2-Methylcyclobutyl, 3-Methylcyclobutyl, 1-Ethylcyclobutyl, 2-Ethylcyclobutyl, 1,2-Dimethylcyclobutyl, 2,2-Dimethylcyclobutyl, 3,3-Dimethylcyclobutyl, Cyclopentyl, 1-Methylcyclopentyl, 2-Methylcyclopentyl, Cyclohexyl und Cycloheptyl in Betracht.

Die unsubstituierten und auch die substituierten Cycloalkylreste für $R^2$ enthalten vorzugsweise 3 bis 6 C-Atome im Ring.

Von den Alkenylresten mit 3 bis 8 C-Atomen, die für $R^2$ stehen können, seien Allyl, But-2-enyl, But-3-enyl, 1-Methylallyl, 2-Methylallyl und Pent-4-enyl genannt.

Bevorzugte Alkenylreste für $R^2$ sind solche mit 3 bis 5 C-Atomen.

Alkinylreste mit 3 bis 8 C-Atomen für $R^2$ sind beispielsweise

Prop-2-inyl, But-2-inyl, But-3-inyl, 1-Methylprop-2-inyl, Pent-2-inyl, Pent-4-inyl, 1-Methylbut-2-inyl, 1-Methylbut-3-inyl und 1,1-Dimethylprop-2-inyl.

Die bevorzugten Alkinylreste für $R^2$ sind solche mit 3 bis 5 C-Atomen.

Beispiele für die ein bis drei gleiche oder verschiedene Substituenten aus der Gruppe Alkyl mit 1 bis 4 C-Atomen, wie Methyl, Ethyl oder Propyl, Alkoxy mit 1 bis 4 C-Atomen im Alkyl, wie Methoxy oder Ethoxy, Halogen, wie Chlor, Brom oder Fluor, Trifluormethyl, Cyan oder Nitro tragende Phenylgruppe, die für $R^2$ stehen kann, sind

o-Tolyl, m-Tolyl, p-Tolyl, p-Ethylphenyl, o,p-Dimethylphenyl, o,m,p-Trimethylphenyl, o-Methoxyphenyl, m-Methoxyphenyl, p-Methoxyphenyl, o-Ethoxyphenyl,

o-Ethoxy-p-ethylphenyl, o-Chlorphenyl, m-Chlorphenyl, p-Chlorphenyl, p-Bromphenyl, p-Fluorphenyl, m,p-Dichlorphenyl, p-Chlor-o-methylphenyl, m-Trifluormethylphenyl, p-Cyanphenyl, o-Nitrophenyl, m-Nitrophenyl und p-Nitrophenyl.

Im Hinblick auf $R^1$ sind bevorzugte Verbindungen die, in denen $R^1$ für eine Methylgruppe steht.

Die Dihydropyridazinone der Formel I werden hergestellt, indem man ein Aminophenyl-dihydropyridazinon der Formel II,

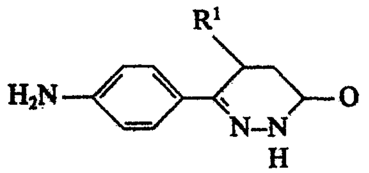

(II)

in der $R^1$ die für Formel I angegebenen Bedeutungen hat, mit einer Verbindung der Formel III,

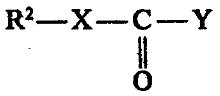

(III)

in der X und $R^2$ die für Formel I angegebenen Bedeutungen haben und für Y ein Halogenatom, insbesondere Chlor, steht, in an sich bekannter Weise umsetzt.

Diese Umsetzung wird unter an sich üblichen Bedingungen durchgeführt. In der Regel unter Verwendung von wenigstens einer äquimolaren Menge des Halogenameisensäureesters oder des Halogenthioameisensäure-S-esters der Formel III, zweckmäßig in Gegenwart eines Lösungsmittels und gegebenenfalls in Gegenwart einer Hilfsbase als säurebindendes Mittel und bei Temperaturen zwische 0 und 140, vorzugsweise 10 bis 100° C, gegebenenfalls bei den Siedetemperaturen des Reaktionsgemisches und gegebenenfalls unter Anwendung von Druck.

Als Lösungsmittel kommen unter den Reaktionsbedingungen inerte Lösungsmittel, wie aromatische Kohlenwasserstoffe, beispielsweise Benzol, Toluol oder Xylol, cyclische aliphatische Ether, wie Tetrahydrofuran oder Dioxan, oder Dialkylformamide, wie Dimethylformamid, in Betracht. Hilfsbasen als säurebindende Mittel sind zweckmäßigerweise anorganische Basen, wie Natrium- oder Kaliumcarbonat, Natrium- oder Kaliumhydrogencarbonat, oder tertiäre organische Amine, wie Triäthylamin.

Gemäß einer weiteren Herstellung werden die neuen Dihydropyridazinone der Formel I erhalten, indem man eine Aminosäure der Formel IV,

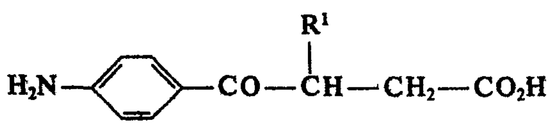

(IV)

in der $R^1$ die für Formel I angegebenen Bedeutungen hat, mit einer Verbindung der Formel III,

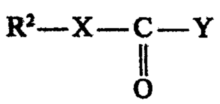

(III)

in der $R^2$, X und Y die oben angegebenen Bedeutungen haben, umsetzt und die dabei entstandene Verbindung der Formel V

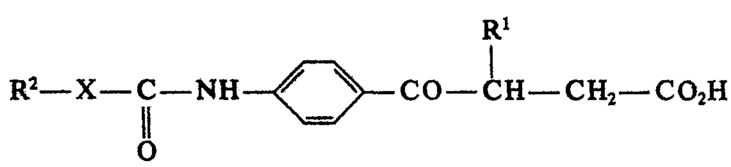

(V)

mit Hydrazin cyclisiert.

Zur Herstellung der Verbindungen V aus den Aminosäuren IV und den Halogenameisensäureestern oder Halogenthioameisensäure-S-estern III wird unter den oben für die Umsetzung eines Aminophenyl-dihydropyridazinons der Formel II mit einer Verbindung der Formel III angegebenen Bedingungen gearbeitet.

Die Cyclisierung einer Verbindung der Formel V mit Hydrazin, das bevorzugt als Hydrat eingesetzt wird, erfolgt vorteilhaft in einem unter den Reaktionsbedingungen inerten Lösungsmittel, insbesondere einem niederen Alkohol, wie Methanol, Ethanol oder Propanol, einem cyclischen aliphatischen Ether, wie Tetrahydrofuran oder Dioxan, oder einem Dialkylformamid, wie Dimethylformamid, und bei Temperaturen von 60 bis 140° C, vorzugsweise 80 bis 120° C. In der Regel werden hierbei pro Mol einer Verbindung der Formel V 1 bis 1,2 Mol Hydrazin verwendet.

Weiterhin kann man die Verbindungen der Formel I herstellen, indem man ein Isocyanat der Formel VI,

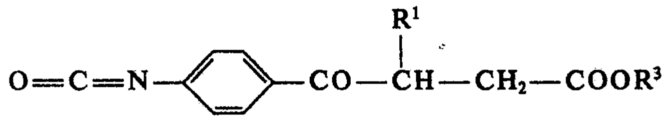

(VI)

in der $R^1$ die für Formel I angegebenen Bedeutungen hat und $R^3$ für einen Alkylrest mit 1 bis 3 C-Atomen, wie Methyl, Ethyl oder Propyl, vorzugsweise Methyl oder Ethyl, steht, mit einer Verbindung der Formel VII,

$$R^2-X-H \qquad \text{(VII)}$$

in der $R^2$ und X die für Formel I angegebenen Bedeutungen haben, umsetzt und die dabei erhaltene Verbindung der Formel VIII

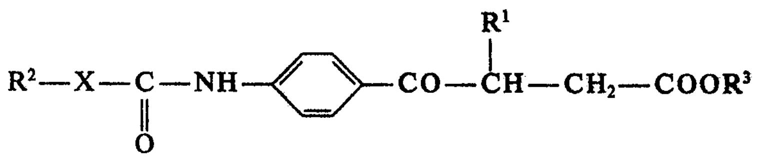

(VIII)

mit Hydrazin cyclisiert.

Die Umsetzung eines Isocyanats der Formel VI mit einer Verbindung der Formel VII wird unter an sich üblichen Bedingungen durchgeführt. In der Regel unter Verwendung von wenigstens einer äquimolaren Menge einer Verbindung der Formel VII, zweckmäßig in Gegenwart eines Lösungsmittels und gegebenenfalls unter Zusatz einer der zur Beschleunigung von Isocyanatreaktionen üblicherweise eingesetzten Katalysatoren bei Temperaturen zwischen 0 und 140, vorzugsweise 20 bis 120° C, gegebenenfalls bei den Siedetemperaturen des Reaktionsgemisches und gegebenenfalls unter Anwendung von Druck.

Als Lösungsmittel kommen unter den Reaktionsbedingungen inerte Lösungsmittel, wie aromatische Kohlenwasserstoffe, beispielsweise Benzol, Toluol oder Xylol, aliphatische oder aromatische chlorierte Kohlenwasserstoffe, zum Beispiel Methylenchlorid oder Chlorbenzol, cyclische aliphatische Ether, wie Tetrahydrofuran oder Dioxan, oder Dialkylformamide, wie Dimethylformamid, in Betracht. Ist die Verbindung der Formel VII flüssig, so kann die Reaktion auch in überschüssigem VII als Lösungsmittel durchgeführt werden.

Geeignete Katalysatoren der Reaktion eines Isocyanats der Formel VI mit einer Verbindung der Formel VII sind beispielsweise anorganische Basen, wie Natrium- oder Kaliumcarbonat, Natrium- oder Kaliumhydrogencarbonat, Alkoholate, wie Natriummethylat oder Kaliumtertiärbutylat, tertiäre organische Amine, wie Triethylamin, Pyridin oder 1,4-Diazabicyclo[2.2]octan, oder Metallverbindungen, zum Beispiel Blei-(IV)-, Zinn-(II)-, Zinn-(IV)- oder Quecksilber-(II)-Verbindungen. Von diesen Metallverbindungen als Katalysatoren sind Zinn-(II)-, Zinn-(IV)- und Quecksilber-(II)-Verbindungen, wie Zinnoctanoat, Dibutylzinndiacetat, Dibutylzinndilaurat oder Phenylquecksilberacetat, bevorzugt.

Die als Ausgangssubstanzen verwendeten Verbindungen der Formel II und auch der Formel IV sind bekannt oder können unter den beispielsweise in den DE-OS 1 670 158 und 2 150 436 oder den US-PS 3 824 271 und 3 888 901 beschriebenen Bedingungen hergestellt werden.

Ausgehend von den Aminosäuren der Formel IV können mittels den in der DE-OS 2 157 453 beschriebenen Synthese (Esterbildung gefolgt durch die Umsetzung mit Phosgen) die Isocyanate der Formel VI hergestellt werden.

Es wird darauf hingewiesen, daß die Verbindungen der Formel I ein asymmetrisches Kohlenstoffatom in 5-Stellung aufweisen und als Racemate erhalten werden. Die vorliegende Erfindung soll die Enantiomeren mit einschließen. Ist eine Trennung erwünscht, so wird diese vorteilhaft auf der Stufe einer Verbindung der Formel II nach bekannten Methoden, z. B. Bildung diastereomerer Salze mit optisch aktiven Hilfssäuren, wie Dibenzoylweinsäure oder Campher-10-sulfonsäure, durchgeführt.

Bei geeignetem Rest $R^2$, z. B. 2-Methylcyclopropyl, tritt ferner in den Verbindungen der Formel I eine geometrische (cis-trans) Isomerie auf. Die vorliegende Erfindung betrifft die jeweiligen cis- und trans-Isomeren und deren Gemische.

Die erfindungsgemäßen Dihydropyridazinone der Formel I zeichnen sich durch thrombozytenaggregationshemmende und blutdrucksenkende Eigenschaften aus. Sie sind als Antihypertensiva und zur Prophylaxe und Therapie thrombo-embolischer Erkrankungen geeignet.

Die vorteilhafte thrombozytenaggregationshemmende Wirkung kann im Vergleich zu Acetylsalicylsäure z. B. an der durch Collagen induzierten Aggregation von Thrombozyten des Menschen festgestellt werden. Zum Nachweis der blutdrucksenkenden Wirkung wurden z. B. Ratten in Urethan-Narkose verwendet. Als Referenzsubstanz dient hier beispielsweise Dihydralazin.

Im einzelnen wurden zur Untersuchung der pharmakodynamischen Eigenschaften folgende Methoden verwendet:

1. Hemmung der durch Collagen induzierten Aggregation von Thrombozyten der Ratte ex vivo. Die Substanzen werden Gruppen von 10 bis 15 männlichen Sprague-Dawley-Ratten (200 bis 250 g) oral appliziert. 1 Stunde nach der Applikation wird in Äther-Narkose Blut entnommen und durch Zentrifugation (300 g, 10 min Dauer bei 4° C) thrombozytenreiches Plasma gewonnen. Die photometrische Messung der Thrombozytenaggregation erfolgt unter Zusatz von Magnesiumchlorid (Endkonzentration 10 mmol/1) und von Collagen Stago (Endkonzentration 0,02 mg/ml) im Born-Aggregometer Mk 3. Als Aggregationsmaß findet die maximale Extinktionsänderung/sec Verwendung. Als ED 33% wirdd die Dosis bestimmt, welche die durch Collagen induzierte Thrombozytenaggregation um 33% hemmt.
2. Antihypertensive Wirkung an der spontan hypertonen Ratte. Die Substanzen werden männlichen spontan hypertonen Okamoto-Ratten (4 bis 8 Tiere/Dosis, Gewicht: 270 bis 360 g) oral appliziert. Der systolische Blutdruck wird vor und 2 Stunden nach der Applikation unblutig am Rattenschwanz mit Hilfe von Piezokristallaufnehmern ermittelt. Als ED 20% wird unter Berücksichtigung der Werte unbehandelter Kontrolltiere die Dosis bestimmt, welche den systolischen Druck um 20% senkt.

Die erhaltenen Ergebnisse sind in der folgenden Tabelle 1 zusammengestellt, wobei zur Erläuterung ausgeführt wird:

Als Vergleichssubstanzen mit der relativen Wirksamkeit (R. W.) von 1 dienen die anerkannten Mittel Acetylsalicylsäure (ASS) und Dihydralazin. Weiterhin wurde verglichen mit einer strukturell ähnlichen Verbindung aus dem Stand der Technik, 6-[p-(2-Chlorpropionylamino)-phenyl]-4,5-dihydro-5-methyl-3(2 H)-pyridazinon (Verbindung $V_1$), die sich bereits als recht wirksam erwiesen hat.

Die gefundenen Zahlenwerte zeigen, daß alle in der Tabelle aufgeführten erfindungsgemäßen Verbindungen im Hinblick auf die sich im Handel befindlichen Verbindungen wenigstens in einer der untersuchten Wirkungen eine wesentlich höhere Wirkung aufweisen.

Tabelle 1

| Verbindung | Thrombozytenaggr. – Hemmung | | Antihypertensive Wirkung | |
|---|---|---|---|---|
| Beispiel Nr. | ED 33% | R. W. | ED 20% | R. W. |
| 1 | 0,532 | 437,97 | 0,793 | 8,64 |
| 2 | 1,16 | 200,86 | 0,285 | 24,03 |
| 3 | 2,97 | 78,45 | 0,382 | 17,93 |
| 7 | 0,140 | 1664,29 | 1,0 | 6,85 |
| 12 | 0,182 | 1280,23 | 0,548 | 12,5 |
| 20 | 0,850 | 274,12 | 2,15 | 3,19 |
| 32 | 13,8 | 16,88 | 0,265 | 25,85 |
| $V_1$ | 0,82 | 284,15 | 1,16 | 5,91 |
| ASS | 233 | 1,00 | – | – |
| Dihydralazin | – | – | 6,85 | 1,00 |

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen zu therapeutischen Zwekken, insbesondere bei der Behandlung von hohem Blutdruck und thrombo-embolischen Erkrankungen.

Die therapeutischen Mittel oder Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt. Dabei kommen beim Menschen Dosen von 1 bis 100 mg, bevorzugt 5 bis 50 mg, in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Maisstärke, Alginsäure oder Polyvinylpyrrolidon, Bindemitteln, wie Stärke oder Gelatine, Gleitmitteln, wie Magnesiumstearat oder Talkum und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen (vgl. L. G. Godman, A. Gilman, The Pharmacological Basis of Therapeutics).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Die Herstellung der neuen 6-Aryl-4,5-dihydro-3(2 H)-pyridazinone wird durch die folgenden Beispiele näher erläutert.

## Beispiel 1

6,0 g (29,5 mmol) 6-(p-Aminophenyl)-4,5-dihydro-5-methyl-3(2 H)-pyridazinon werden mit 3,3 g (34,9 mmol) Chlorameisensäuremethylester in 100 ml absolutem Toluol 6 Stunden bei 80° C gehalten. Man saugt bei 10° C ab, wäscht zuerst mit Toluol, dann mit Wasser und kristallisiert aus Dimethylformamid/Wasser um. Man erhält 3,1 g (40% der Theorie) 4,5-Dihydro-6-(p-methoxycarbonylaminophenyl)-5-methyl-3(2 H)-pyridazinon, farblose Kristalle, Schmelzpunkt 228 bis 229° C.

Analyse für $C_{13}H_{15}N_3O_3$:

| | | | |
|---|---|---|---|
| ber.: | C 59,8 | H 5,8 | N 16,1% |
| gef.: | C 59,8 | H 5,7 | N 16,3%. |

## Beispiel 2

Wird im Beispiel 1 anstatt Chlorameisensäuremethylester Chlorameisensäureethylester (3,8 g (35,0 mmol)) eingesetzt, so erhält man nach der Umkristallisation aus Dimethylformamid/Wasser 3,5 g (43% der Theorie) 4,5-Dihydro-6-(p-ethoxycarbonylaminophenyl)-5-methyl-3(2 H)-pyridazinon, farblose Kristalle, Schmelzpunkt 219 bis 221° C.

Analyse für $C_{14}H_{17}N_3O_3$:

| | | | |
|---|---|---|---|
| ber.: | C 61,1 | H 6,2 | N 15,3% |
| gef.: | C 61,0 | H 6,3 | N 15, 5%. |

## Beispiel 3

Wird das Beispiel 1 mit Chlorameisensäurepropylester (5,45 g (44,5 mmol)) anstelle von Chlorameisensäuremethylester durchgeführt, so erhält man nach dem Umkristallisieren aus Dimethylformamid/Wasser 3,5 g (41% der Theorie) 4,5-Dihydro-5-methyl-6-(p-propoxycarbonylaminophenyl)-3(2 H)-pyridazinon, hellgelbe Kristalle, Schmelzpunkt 207 bis 208° C.

Analyse für $C_{15}H_{19}N_3O_3$:

| | | | |
|---|---|---|---|
| ber.: | C 62,3 | H 6,6 | N 14,5% |
| gef.: | C 62,6 | H 6,4 | N 14,8%. |

Beispiel 4

6,0 g (29,5 mmol) 6-(p-Aminophenyl)-4,5-dihydro-5-methyl-3(2 H)-pyridazinon werden mit 4,0 g (32,6 mmol) Chlorameisensäureisopropylester in 100 ml absolutem Toluol 16 Stunden am Rückfluß gehalten. Man saugt bei 10° C ab, wäscht zuerst mit Toluol, denn mit Wasser und kristallisiert zweimal aus Dimethylformamid/Wasser um. Man erhält 4,3 g (50% der Theorie) 4,5-Dihydro-6-(p-isopropoxycarbonyl-aminophenyl)-5-methyl-3(2 H)-pyridazinon, farblose Kristalle, Schmelzpunkt 236 bis 237° C.

Analyse für $C_{15}H_{19}N_3O_3$:

| | | | |
|---|---|---|---|
| ber.: | C 62,3 | H 6,6 | N 14,5% |
| gef.: | C 62,3 | H 6,7 | N 14,8%. |

Beispiel 5

Führt man das Beispiel 1 unter Verwendung von Chlorameisensäurebutylester (4,45 g (32,6 mmol)) anstelle von Chlorameisensäuremethylester durch, so erhält man nach zweimaliger Umkristallisation aus Ethanol/Wasser 3,0 g (33% der Theorie) 6-(p-Butoxycarbonylaminophenyl)-4,5-dihydro-5-methyl-3(2 H)-pyridazinon, farblose Kristalle, Schmelzpunkt 203 bis 204° C.

Analyse für $C_{16}H_{21}N_3O_3$:

| | | | |
|---|---|---|---|
| ber.: | C 63,3 | H 7,0 | N 13,9% |
| gef.: | C 63,0 | H 6,9 | N 14,0%. |

Beispiel 6

Führt man das Beispiel 1 unter Verwendung von Chlorameisensäureisobutylester (4,45 g (32,6 mmol)) anstelle von Chlorameisensäuremethylester durch, so erhält man nach der Umkristallisation aus Ethanol/Wasser 3,6 g (40% der Theorie) 4,5-Dihydro-6-(p-isobutoxycarbonylaminophenyl)-5-methyl-3(2 H)-pyridazinon, farblose Kristalle, Schmelzpunkt 186 bis 187° C.

Analyse für $C_{16}H_{21}N_3O_3$:

| | | | |
|---|---|---|---|
| ber.: | C 63,3 | H 7,0 | N 13,9% |
| gef.: | C 63,5 | H 6,8 | N 14,1%. |

Beispiel 7

Führt man das Beispiel 1 unter Verwendung von Chlorameisensäure-2-chlorethylester (4,65 g (32,5 mmol)) anstelle von Chlorameisensäuremethylester durch, so erhält man nach der Umkristallisation aus Ethanol/Wasser 3,6 g (39% der Theorie) 6-[p-(2-Chlorethoxycarbonylamino)phenyl]-4,5-dihydro-5-methyl-3(2 H)-pyridazinon, fast farblose Kristalle, Schmelzpunkt 206 bis 207° C.

Analyse für $C_{14}H_{16}ClN_3O_3$:

| | | | | |
|---|---|---|---|---|
| ber. : | C 54,3 | H 5,2 | Cl 11,4 | N 13,6% |
| gef.: | C 54,6 | H 5,3 | Cl 11,4 | N 13,8%. |

Beispiel 8

Wird das Beispiel 1 mit Chlorameisensäure-2-bromethylester (6,1 g (32,5 mmol) anstelle von Chlorameisensäuremethylester durchgeführt, so erhält man nach zweimaliger Umkristallisation aus Methanol/Wasser 2,4 g (23% der Theorie) 6-[p-(2-Bromethoxycarbonylamino)phenyl]-4,5-dihydro-5-methyl-3(2 H)-pyridazinon, hellgelbe Kristalle, Schmelzpunkt 218 bis 220° C.

Analyse für $C_{14}H_{16}BrN_3O_3$:

| | | | | |
|---|---|---|---|---|
| ber.: | C 47,5 | H 4,6 | Br 22,6 | N 11,9% |
| gef.: | C 48,0 | H 4,7 | Br 21,5 | N 12,0%. |

## Beispiel 9

Führt man das Beispiel 1 unter Verwendung von Chlorameisensäure-2,2,2-trichlorethylester (6,9 g (32,6 mmol)) anstelle von Chlorameisensäuremethylester durch, so erhält man nach zweimaliger Umkristallisation aus Ethanol/Wasser 3,8 g (34% der Theorie) 4,5-Dihydro-5-methyl-6-[p-(2,2,2-trichlorethoxycarbonylamino)phenyl]-3(2 H)-pyridazinon, farblose Kristalle, Schmelzpunkt 211 bis 212° C.

Analyse für $C_{14}H_{14}Cl_3N_3O_3$:

| | | | | |
|---|---|---|---|---|
| ber.: | C 44,4 | H 3,7 | Cl 28,1 | N 11,1% |
| gef.: | C 44,9 | H 3,8 | Cl 27,9 | N 11,3%. |

## Beispiel 10

Führt man das Beispiel 1 unter Verwendung von Chlorameisensäure-2-methoxyethylester (6,2 g (44,7 mmol)) anstelle von Chlorameisensäuremethylester durch, so isoliert man nach zweimaliger Umkristallisation aus Methanol 1,7 g (19% der Theorie) 4,5-Dihydro-6-[p-(2-methoxyethoxycarbonylamino)-phenyl]-5-methyl-3(2 H)-pyridazinon, gelbliche Kristalle, Schmelzpunkt 216 bis 217° C.

Analyse für $C_{15}H_{19}N_3O_4$:

| | | | |
|---|---|---|---|
| ber.: | C 59,0 | H 6,3 | N 13,8% |
| gef.: | C 58,9 | H 6,2 | N 14,1%. |

## Beispiel 11

Zu einer Lösung von 8,8 g (89,0 mmol) Phosgen in 50 ml absolutem Ether tropft man unter Rühren bei 10 bis 15° C 3,2 g (44,4 mmol) Hydroxymethyl)cyclopropan, gelöst in 50 ml absolutem Ether. Anschließend wird die Reaktionslösung noch 3 Stunden bei Raumtemperatur nachgerührt. Man entfernt nun zuerst den Überschuß des Phosgens durch einen trockenen Strom von Stickstoff und hernach unter vermindertem Druck bei Raumtemperatur den Ether. Der zurückbleibende Chlorameisensäurecyclopropylmethylester wird mit 6,0 g (29,5 mmol) 6-(p-Aminophenyl)-4,5-dihydro-5-methyl-3(2 H)-pyridazinon und 100 ml absolutem Toluol 7 Stunden bei 80° C gehalten. Man saugt bei 10° C ab, wäscht zuerst mit Toluol, dann mit Wasser und kristallisiert zweimal aus Dimethylformamid/Wasser um. Man isoliert 3,0 g (34% der Theorie) 6-(p-Cyclopropylmethoxycarbonylaminophenyl)-4,5-dihydro-5-methyl-3(2 H)-pyridazinon, farblose Kristalle, Schmelzpunkt 240 bis 241° C.

Analyse für $C_{16}H_{19}N_3O_3$:

| | | | |
|---|---|---|---|
| ber.: | C 63,8 | H 6,4 | N 13,9% |
| gef.: | C 63,6 | H 6,2 | N 14,2%. |

## Beispiel 12

Führt man das Bespiel 1 unter Verwendung von Chlorameisensäurebenzylester (5,5 g (32,2 mmol)) anstelle von Chlorameisensäuremethylester durch, so erhält man nach dem Umkristallisieren aus Ethanol/Wasser 4,2 g (42% der Theorie) 6-(p-Benzyloxycarbonylaminophenyl)-4,5-dihydro-5-methyl-3(2 H)-pyridazinon, farblose Kristalle, Schmelzpunkt 168 bis 169° C.

Analyse für $C_{19}H_{19}N_3O_3$:

| | | | |
|---|---|---|---|
| ber.: | C 67,6 | H 5,7 | N 12,5% |
| gef.: | C 67,6 | H 5,7 | N 12,7%. |

## Beispiel 13

Zu einer Lösung von 11,5 g (116,3 mmol) Phosgen in 100 ml absolutem Ether gibt man tropfenweise unter Rühren bei 10 bis 15° C 5,0 g (69,3 mmol) Cyclobutanol, gelöst in 80 ml absolutem Ether. Anschließend wird die Reaktionslösung noch 3 Stunden bei Raumtemperatur nachgerührt. Man entfernt nun zuerst den Überschuß des Phosgens durch einen trockenen Strom von Stickstoff und hernach unter vermindertem Druck bei Raumtemperatur den Ether. Der zurückbleibende Chlorameisensäurecyclobutylester wird mit 6,0 g (29,5 mmol) 6-(p-Aminophenyl)-4,5-dihydro-5-methyl-3(2 H)-pyridazinon und 180 ml absolutem Tetrahydrofuran zuerst 20 Stunden bei Raumtemperatur und dann noch 4 Stunden bei 40 bis 45° C gehalten. Man saugt bei 10° C ab, wäscht zuerst mit Tetrahydrofuran, dann mit Wasser und kristallisiert aus Dimethylformamid/Wasser um. Man isoliert 2,8 g (31% der Theorie) 6-(p-Cyclobu-

tyloxycarbonylaminophenyl)-4,5-dihydro-5-methyl-3(2 H)-pyridazinon, farblose Kristalle, Schmelzpunkt 245 bis 246° C.

Analyse für $C_{16}H_{19}N_3O_3$:

| | | | |
|---|---|---|---|
| ber.: | C 63,8 | H 6,4 | N 13,9% |
| gef.: | C 63,4 | H 6,3 | N 14,2%. |

Beispiel 14

Wird im Beispiel 1 anstatt Chlorameisensäuremethylester Chlorameisensäurecyclohexylester (7,2 g (44,3 mmol)) eingesetzt, so erhält man nach zweimaliger Umkristallisation aus Dimethylformamid/Wasser 3,0 g (31% der Theorie) 6-(p-Cyclohexyloxycarbonylaminophenyl)-4,5-dihydro-5-methyl-3(2 H)-pyridazinon, fast farblose Kristalle, Schmelzpunkt 194 bis 195° C.

Analyse für $C_{18}H_{23}N_3O_3$:

| | | | |
|---|---|---|---|
| ber.: | C 65,6 | H 7,0 | N 12,8% |
| gef.: | C 65,3 | H 7,1 | N 13,1%. |

Beispiel 15

Führt man das Beispiel 1 unter Verwendung von Chlorameisensäureallylester (3,9 g (32,3 mmol)) anstelle von Chlorameisensäuremethylester durch, so erhält man nach dem Umkristallisieren aus Methanol 3,0 g (35% der Theorie) 6-(p-Allyloxycarbonylaminophenyl)-4,5-dihydro-5-methyl-3(2 H)-pyridazinon, hellgelbe Kristalle, Schmelzpunkt 206 bis 207° C.

Analyse für $C_{15}H_{17}N_3O_3$:

| | | | |
|---|---|---|---|
| ber.: | C 62,7 | H 6,0 | N 14,6% |
| gef.: | C 62,5 | H 5,9 | N 14,9%. |

Beispiel 16

Führt man das Beispiel 1 unter Verwendung von Chlorameisensäurephenylester (5,0 g (31,9 mmol)) anstelle von Chlorameisensäuremethylester durch, so isoliert man nach dem Umkristallisieren aus Dimethylformamid/Wasser 3,9 g (41% der Theorie) 4,5-Dihydro-5-methyl-6-(p-phenoxycarbonylaminophenyl)-3(2 H)-pyridazinon, fast farblose Kristalle, Schmelzpunkt 200 bis 202° C.

Analyse für $C_{18}H_{17}N_3O_3$:

| | | | |
|---|---|---|---|
| ber.: | C 66,9 | H 5,3 | N 13,0% |
| gef.: | C 66,9 | H 5,5 | N 13,2%. |

Beispiel 17

5,0 g (24,6 mmol) 6-(p-Aminophenyl)-4,5-dihydro-5-methyl-3(2 H)-pyridazinon werden mit 5,05 g (29,6 mmol) Chlorameisensäure-m-tolylester in 100 ml absolutem Toluol 6 Stunden bei 80° C gehalten. Man saugt bei 10° C ab, wäscht zuerst mit Toluol, dann mit Wasser und kristallisiert zweimal aus Dimethylformamid/Wasser um. Man isoliert 2,5 g (30% der Theorie) 4,5-Dihydro-5-methyl-6-[p-(m-tolyloxycarbonylamino)phenyl]-3(2 H)-pyridazinon-viertelhydrat, fast farblose Kristalle, Schmelzpunkt 166 bis 167° C.

Analyse für $C_{19}H_{19}N_3O_3 \cdot 1/4\ H_2O$:

| | | | | |
|---|---|---|---|---|
| ber.: | C 66,8 | H 5,7 | N 12,3 | O 15,2% |
| gef.: | C 66,6 | H 5,7 | N 12,2 | O 15,0%. |

Beispiel 18

Wird im Beispiel 17 anstatt Chlorameisensäure-m-tolylester Chlorameisensäure-o-methoxyphenylester (5,5 g (29,5 mmol)) eingesetzt, so isoliert man nach dem Umkristallisieren aus Dimethylformamid/Wasser 3,0 g (35% der Theorie) 4,5-Dihydro-6-[p-(o-methoxyphenoxycarbonylamino)phenyl]-5-methyl-3(2 H)-pyridazinon, hellgelbe Kristalle, Schmelzpunkt 185 bis 186° C.

Analyse für $C_{19}H_{19}N_3O_4$:

| | | | |
|---|---|---|---|
| ber.: | C 64,6 | H 5,4 | N 11,9% |
| gef.: | C 64,6 | H 5,3 | N 12,2%. |

Beispiel 19

5,0 g (24,6 mmol) 6-(p-Aminophenyl)-4,5-dihydro-5-methyl-3(2 H)-pyridazinon werden unter Rühren in 150 ml absolutem Tetrahydrofuran durch Erwärmen auf 60°C gelöst. Man läßt die Lösung auf Raumtemperatur abkühlen, fügt 5,0 g (24,8 mmol) Chlorameisensäure-p-nitrophenylester hinzu und rührt zuerst 20 Stunden bei Raumtemperatur und dann noch 5 Stunden bei Rückflußtemperatur nach. Man engt auf ca. 70 ml ein, gibt 100 ml Wasser zu und saugt ab. Nach dem Umkristallisieren bei Raumtemperatur aus Dimethylformamid/Wasser isoliert man 6,5 g (72% der Theorie) 4,5-Dihydro-5-methyl-6-[p-(p-nitrophenoxycarbonylamino)phenyl]-3(2 H)-pyridazinon, gelbe Kristalle, Schmelzpunkt 177 bis 178°C.

Analyse für $C_{18}H_{16}N_4O_5$:

| | | | |
|---|---|---|---|
| ber.: | C 58,7 | H 4,4 | N 15,2% |
| gef.: | C 58,5 | H 4,4 | N 15,2%. |

Beispiel 20

Wird im Beispiel 1 anstatt Chlorameisensäuremethylester Chlorthioameisensäure-S-methylester (3,6 g (32,6 mmol)) eingesetzt, so isoliert man nach dem Umkristallisieren aus Ethanol/Wasser 3,1 g (38% der Theorie) 4,5-Dihydro-5-methyl-6-(p-methylmercaptocarbonylaminophenyl)-3(2 H)-pyridazinon, farblose Kristalle, Schmelzpunkt 214 bis 216°C mit Zersetzung.

Analyse für $C_{13}H_{15}N_3O_2S$:

| | | | | |
|---|---|---|---|---|
| ber.: | C 56,3 | H 5,5 | N 15,2 | S 11,6% |
| gef.: | C 56,2 | H 5,4 | N 15,5 | S 11,3%. |

Beispiel 21

Führt man das Beispiel 1 unter Verwendung von Chlorthioameisensäure-S-ethylester (4,15 g (33,3 mmol)) anstelle von Chlorameisensäuremethylester durch, so erhält man nach dem Umkristallisieren aus Essigester 3,6 g (42% der Theorie) 4,5-Dihydro-6-(p-ethylmercaptocarbonylaminophenyl)-5-methyl-3(2 H)-pyridazinon, gelbbraune Kristalle, Schmelzpunkt 212 bis 213°C mit Zersetzung.

Analyse für $C_{14}H_{17}N_3O_2S$:

| | | | | |
|---|---|---|---|---|
| ber.: | C 57,7 | H 5,9 | N 14,4 | S 11,0% |
| gef.: | C 57,8 | H 6,0 | N 14,3 | S 10,5%. |

Beispiel 22

Wird im Beispiel 1 anstatt Chlorameisensäuremethylester Chlorthioameisensäure-S-propylester (4,5 g (32, 5 mmol)) eingesetzt, so erhält man nach dem Umkristallisieren aus Dimethylformamid/Wasser 2,8 g (31% der Theorie) 4,5-Dihydro-5-methyl-6-(p-propylmercaptocarbonylaminophenyl)-3(2 H)-pyridazinon, farblose Kristalle, Schmelzpunkt 195 bis 196°C.

Analyse für $C_{15}H_{19}N_3O_2S$:

| | | | | |
|---|---|---|---|---|
| ber.: | C 59,0 | H 6,3 | N 13,8 | S10,5% |
| gef.: | C 59,3 | H 6,3 | N 14,2 | S 10,0%. |

Beispiel 23

5,0 g (24,6 mmol) 6-(p-Aminophenyl)-4,5-dihydro-5-methyl-3(2 H)-pyridazinon werden unter Rühren in 150 ml absolutem Tetrahydrofuran durch Erwärmen auf 60°C gelöst. Man läßt die Lösung auf Raumtemperatur abkühlen, fügt 5,6 g (36,7 mmol) Chlorameisensäure-2-methoxypropylester hinzu und rührt noch 14 Stunden bei Rückflußtemperatur nach. Man engt ein, gibt Wasser zu und saugt ab. Nach dem Umkristallisieren aus Dimethylformamid/Wasser isoliert man 5,7 g (73% der Theorie)

4,5-Dihydro-6-[p-(2-methoxypropoxycarbonylamino)phenyl]-5-methyl-3(2 H)-pyridazinon, farblose Kristalle, Schmelzpunkt 173 bis 175° C.

Analyse für $C_{16}H_{21}N_3O_4$:

| | | | |
|---|---|---|---|
| ber.: | C 60,2 | H 6,6 | N 13,2% |
| gef.: | C 60,4 | H 6,5 | N 13,4%. |

Beispiel 24

Wird im Beispiel 23 anstatt Chlorameisensäure-2-methoxypropylester Chlorameisensäure-2-methoxyisopropylester eingesetzt, so isoliert man nach dem Umkristallisieren aus Dimethylformamid/Wasser 5,8 g (74% der Theorie) 4,5-Dihydro-6-[p-(2-methoxyisopropoxycarbonylamino)phenyl]-5-methyl-3(2 H)-pyridazinon, farblose Kristalle, Schmelzpunkt 208 bis 210° C.

Analyse für $C_{16}H_{21}N_3O_4$:

| | | | |
|---|---|---|---|
| ber.: | C 60,2 | H 6,6 | N 13,2% |
| gef.: | C 60,4 | H 6,5 | N 13,6%. |

Beispiel 25

Führt man das Beispiel 23 unter Verwendung von Chlorameisensäure-3-ethoxypropylester (6,1 g (36,6 mmol)) anstelle von Chlorameisensäure-2-methoxypropylester durch, so erhält man nach der Umkristallisation aus Dimethylformamid/Wasser 5,8 g (71% der Theorie) 4,5-Dihydro-6-[p-(3-ethoxypropoxycarbonylamino)phenyl]-5-methyl-3(2 H)-pyridazinon, farblose Kristalle, Schmelzpunkt 136 bis 137° C.

Analyse für $C_{17}H_{23}N_3O_4$:

| | | | |
|---|---|---|---|
| ber.: | C 61,2 | H 7,0 | N 12,6% |
| gef.: | C 61,4 | H 6,9 | N 12,6%. |

Beispiel 26

Zu einer Lösung von 10,4 g (105 mmol) Phosgen in 100 ml absolutem Ether gibt man tropfenweise unter Rühren bei 0° C 4,3 g (49,9 mmol) (Hydroxymethyl)cyclobutan. Anschließend wird die Reaktionslösung 1 Stunde bei 0° C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäurecyclobutylmethylester (6,9 g) wird nach der im Beispiel 23 beschriebenen Methode mit 5,0 g (24,6 mmol) 6-(p-Aminophenyl)-4,5-dihydro-5-methyl-3(2 H)-pyridazinon umgesetzt. Die Aufarbeitung des Reaktionsgemisches wird ebenfalls analog Beispiel 23 durchgeführt. Nach dem Umkristallisieren aus Dimethylformamid/Wasser erhält man 5,5 g (71% der Theorie) 6-(p-Cyclobutylmethoxycarbonylaminophenyl)-4,5-dihydro-5-methyl-3(2 H)-pyridazinon, farblose Kristalle, Schmelzpunkt 199 bis 200° C.

Analyse für $C_{17}H_{21}N_3O_3$:

| | | | |
|---|---|---|---|
| ber.: | C 64,8 | H 6,7 | N 13,3% |
| gef.: | C 64,6 | H 6,5 | N 13,5%. |

Beispiel 27

Zu einer Lösung von 13,0 g (131,4 mmol) Phosgen in 100 ml absolutem Ether gibt man tropfenweise unter Rühren bei 0 bis 10° C 5,9 g (51,7 mmol) Hydroxymethyl)cyclohexan. Anschließend wird die Reaktionslösung 1 Stunde bei 0 bis 10° C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäurecyclohexylmethylester (8,5 g) wird analog Beispiel 23 mit 5,0 g (24,6 mmol) 6-(p-Aminophenyl)-4,5-dihydro-5-methyl-3(2 H)-pyridazinon umgesetzt. Die Aufarbeitung des Reaktionsgemisches erfolgt ebenfalls nach der im Beispiel 23 beschriebenen Methode. Nach der Umkristallisation zuerst aus Dimethylformamid/Wasser, dann aus Essigester/Petrolether und schließlich noch aus Aceton/Wasser isoliert man 2,1 g (25% der Theorie) 6-(p-Cyclohexylmethoxycarbonylaminophenyl)-4,5-dihydro-5-methyl-3(2 H)-pyridazinon, farblose Kristalle, Schmelzpunkt 223 bis 225° C.

Analyse für $C_{19}H_{25}N_3O_3$:

| | | | |
|---|---|---|---|
| ber.: | C 66,5 | H 7,3 | N 12,2% |
| gef.: | C 66,4 | H 7,4 | N 12,7%. |

Beispiel 28

Zu einer Lösung von 9,9 g (100 mmol) Phosgen in 100 ml absolutem Ether gibt man tropfenweise unter Rühren bei 0° C 4,3 g (49,9 mmol) 1-Cyclopropylethanol. Anschließend wird die Reaktionslösung 1 Stunde bei 0° C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäure-1-cyclopropylethylester (6,6 g) wird nach der im Beispiel 23 beschriebenen Methode mit 5,0 g (24,6 mmol) 6-(p-Aminophenyl)-4,5-dihydro-5-methyl-3(2 H)-pyridazinon umgesetzt. Die Aufarbeitung des Reaktionsgemisches wird ebenfalls analog Beispiel 23 vorgenommen. Nach dem Umkristallisieren, zuerst aus Dimethylformamid/Wasser und dann noch aus Ethanol/Ether isoliert man 1,3 g (17% der Theorie) 6-[p-(1-Cyclopropylethoxycarbonylamino)phenyl]-4,5-dihydro-5-methyl-3(2 H)-pyridazinon, farblose Kristalle, Schmelzpunkt 223 bis 224° C.

Analyse für $C_{17}H_{21}N_3O_3$:

| | | | |
|---|---|---|---|
| ber.: | C 64,8 | H 6,7 | N 13,3% |
| gef.: | C 64,9 | H 6,7 | N 13,3%. |

Beispiel 29

Zu einer Lösung von 9,8 g (99 mmol) Phosgen in 100 ml absolutem Ether gibt man tropfenweise unter Rühren bei 0 bis 5° C 6,1 g (49,9 mmol) 2-Phenylethanol. Anschließend wird die Reaktionslösung 1 Stunde bei 5 bis 10° C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäure-2-phenylethylester (9,1g) wird nach der im Beispiel 23 beschriebenen Methode mit 5,0 g (24,6 mmol) 6-(p-Aminophenyl)-4,5-dihydro-5-methyl-3(2 H)-pyridazinon umgesetzt. Aufgearbeitet wird ebenfalls analog Beispiel 23. Nach dem Umkristallisieren aus Essigester/Petrolether erhält man 4,7 g (54% der Theorie) 4,5-Dihydro-5-methyl-6-[p-(2-phenylethoxycarbonylamino)phenyl]-3(2 H)-pyridazinon, farblose Kristalle, Schmelzpunkt 163 bis 164° C.

Analyse für $C_{20}H_{21}N_3O_3$:

| | | | |
|---|---|---|---|
| ber.: | C 68,4 | H 6,0 | N 12,0% |
| gef.: | C 68,1 | H 6,1 | N 12,1%. |

Beispiel 30

Zu einer Lösung von 9,9 g (100 mmol) Phosgen in 100 ml absolutem Ether gibt man tropfenweise unter Rühren bei —20° C 6,9 g (49,9 mmol) m-Methoxybenzylalkohol. Anschließend wird die Reaktionslösung 1 Stunde bei —10° C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäure-m-methoxybenzylester (11 g) wird nach der im Beispiel 23 beschriebenen Methode mit 5,0 g (24,6 mmol) 6-(p-Aminophenyl)-4,5-dihydro-5-methyl-3(2 H)-pyridazinon umgesetzt. Hier wird jedoch nur 8 Stunden bei Rückflußtemperatur nachgerührt. Aufgearbeitet wird analog Beispiel 23. Nach der Umkristallisation aus Propanol erhält man 7,3 g (81% der Theorie) 4,5-Dihydro-6-[p-(m-methoxybenzyloxycarbonylamino)phenyl]-5-methyl-3(2 H)-pyridazinon, farblose Kristalle, Schmelzpunkt 199 bis 200° C.

Analyse für $C_{20}H_{21}N_3O_4$:

| | | | |
|---|---|---|---|
| ber.: | C 65,4 | H 5,8 | N 11,4% |
| gef.: | C 65,4 | H 5,9 | N 11,4%. |

Beispiel 31

Zu einer Lösung von 11,2 g (113 mmol) Phosgen in 100 ml absolutem Ether gibt man tropfenweise unter Rühren bei —20° C eine Lösung von 7,1 g (49,8 mmol) m-Chlorbenzylalkohol in 50 ml absolutem

Ether. Anschließend wird die Reaktionslösung 1 Stunde bei −10°C gerührt. Man rührt eine weitere Stunde bei Raumtemperatur und entfernt dann zuerst den Überschuß des Phosgens durch einen trockenen Strom von Stickstoff und hernach unter vermindertem Druck bei Raumtemperatur den Ether. Der zurückbleibende Chlorameisensäure-m-chlorbenzylester (12,0 g) wird in 50 ml absolutem Tetrahydrofuran gelöst und bei Raumtemperatur zu einer nach der im Beispiel 23 beschriebenen Methode hergestellten Lösung von 5,0 g (24,6 mmol) 6-(p-Aminophenyl)-4,5-dihydro-5-methyl-3(2 H)-pyridazinon in 100 ml absolutem Tetrahydrofuran gegeben. Anschließend wird das Reaktionsgemisch 14 Stunden bei Rückflußtemperatur gehalten. Aufgearbeitet wird analog Beispiel 23. Nach dem Umkristallisieren, zuerst aus Chloroform/Petrolether, dann aus Aceton/Wasser und schließlich noch aus Propanol/Wasser isoliert man 4,0 g (44% der Theorie) 6-[p-(m-Chlorbenzyloxycarbonylamino)phenyl]-4,5-dihydro-5-methyl-3(2 H)-pyridazinon, farblose Kristalle, Schmelzpunkt 165 bis 166° C.

Analyse für $C_{19}H_{18}ClN_3O_3$:

| | | | | |
|---|---|---|---|---|
| ber.: | C 61,4 | H 4,9 | Cl 9,5 | N 11,3% |
| gef.: | C 61,5 | H 5,0 | Cl 9,9 | N 11,5%. |

Beispiel 32

Zu einer Lösung von 9,9 g (100 mmol) Phosgen in 100 ml absolutem Ether gibt man tropfenweise unter Rühren bei −10° C 2,8 g (49,9 mmol) Prop-2-inol. Anschließend wird die Reaktionslösung 1 Stunde bei −10° C gerührt. Man rührt eine weitere Stunde bei Raumtemperatur und entfernt dann zuerst den Überschuß des Phosgens durch einen trockenen Strom von Stickstoff und danach unter vermindertem Druck bei Raumtemperatur den Ether. Der zurückbleibende Chlorameisensäureprop-2-inylester (5,2 g) wird bei Raumtemperatur zu einer nach der im Beispiel 23 beschriebenen Methode hergestellten Lösung von 5,0 g (24,6 mmol) 6-(p-Aminophenyl)-4,5-dihydro-5-methyl-3(2 H)-pyridazinon in 150 ml absolutem Tetrahydrofuran gegeben. Anschließend wird das Reaktionsgemisch 1 Stunde bei Rückflußtemperatur gehalten. Man engt auf circa 70 ml ein, gibt Wasser zu und saugt ab. Nach der Umkristallisation, zuerst aus Ethanol/Wasser, dann aus Aceton/Wasser und schließlich noch aus Ethanol erhält man 2,9 g (41% der Theorie) 4,5-Dihydro-5-methyl-6-[p-(prop-2-inyloxycarbonylamino)phenyl]-3(2 H)-pyridazinon, farblose Kristalle, Schmelzpunkt 204 bis 205° C.

Analyse für $C_{15}H_{15}N_3O_3$:

| | | | |
|---|---|---|---|
| ber.: | C 63,1 | H 5,3 | N 14,7% |
| gef.: | C 63,3 | H 5,4 | N 15,0%. |

Beispiel 33

Zu einer Lösung von 9,9 g (100 mmol) Phosgen in 100 ml absolutem Ether gibt man tropfenweise unter Rühren bei −10° C 3,5 g (49,9 mmol) 1-Methylprop-2-inol. Anschließend wird die Reaktionslösung 1 Stunde bei −10° C gerührt. Man rührt eine weitere Stunde bei Raumtemperatur und entfernt dann zuerst den Überschuß des Phosgens durch einen trockenen Strom von Stickstoff und danach unter vermindertem Druck bei Raumtemperatur den Ether. Der zurückbleibende Chlorameisensäure-1-methylprop-2-inylester (5,9 g) wird nach der im Beispiel 23 beschriebenen Methode mit 5,0 g (24,6 mmol) 6-(p-Aminophenyl)-4,5-dihydro-5-methyl-3(2 H)-pyridazinon umgesetzt. Aufgearbeitet wird ebenfalls analog Beispiel 23. Nach dem Umkristallisieren zuerst aus Ethanol/Wasser, dann aus Essigester/Petrolether und anschließend noch aus Dimethylformamid/Wasser isoliert man 1,7 g (23% der Theorie) 4,5-Dihydro-5-methyl-6-[p-(1-methylprop-2-inyloxycarbonylamino)phenyl]-3(2 H)-pyridazinon, farblose Kristalle, Schmelzpunkt 206 bis 207° C.

Analyse für $C_{16}H_{17}N_3O_3$:

| | | | |
|---|---|---|---|
| ber.: | C 64,2 | H 5,7 | N 14,0% |
| gef.: | C 64,2 | H 5,8 | N 14,2%. |

Formulierungsbeispiele, die in üblicher Weise hergestellt werden:

1. Tabletten:

| | |
|---|---|
| Wirkstoff | 10 mg |
| Polyvinylpyrrolidon (mittl. M. G. 25 000) | 170 mg |
| Polyäthylenglykol (mittl. M. G. 4000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10%iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M. G. 4000), Hydroxypropylmethylcellulose, Talkum und Magnesiumstearat vermischt und zu Tabletten a 240 mg verpreßt.

2. Beispiel für Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8%igen wäßrigen Lösung des Polyvinylpyrrolidons durch Sieb 1,5 mm granuliert, bei 50°C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

**Patentansprüche**

1. Verbindungen der Formel I,

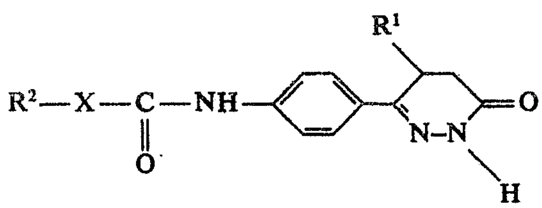

(I)

in der

X ein Sauerstoffatom oder ein Schwefelatom,
$R^1$ einen Alkylrest mit 1 bis 3 C-Atomen und
$R^2$ einen Alkylrest mit 1 bis 8 C-Atomen, der gegebenenfalls durch ein bis vier Halogenatome, eine Alkoxygruppe mit 1 bis 4 C-Atomen im Alkyl, die mindestens durch zwei C-Atome von X getrennt ist, eine Cycloalkylgruppe mit 3 bis 8 C-Atomen im Ring, die gegebenenfalls ein oder zwei Alkylgruppen mit 1 bis 3 C-Atomen aufweist, oder einen Phenylrest, der gegebenenfalls ein bis drei gleiche oder verschiedene Substituenten aus der Gruppe Alkyl mit 1 bis 3 C-Atomen, Alkoxy mit 1 bis 3 C-Atomen im Alkyl, Halogen, Trifluormethyl oder Nitro aufweist, substituiert ist, einen Cycloalkylrest mit 3 bis 8 C-Atomen im Ring, der gegebenenfalls ein- bis vierfach durch Alkylreste mit 1 bis 4 C-Atomen substituiert ist, einen Alkenylrest mit 3 bis 8 C-Atomen, einen Alkinylrest mit 3 bis 8 C-Atomen oder einen Phenylrest, der gegebenenfalls durch ein bis drei gleiche oder verschiedene Substituenten aus der Gruppe Alkyl mit 1 bis 4 C-Atomen, Alkoxy mit 1 bis 4 C-Atomen im Alkyl, Halogen, Trifluormethyl, Cyan oder Nitro substituiert ist,

bedeuten.

2. Verbindungen der Formel I nach Anspruch 1, in denen $R^1$ Methyl und $R^2$ einen Alkylrest mit 1 bis 4 C-Atomen, einen Alkylrest mit 1 bis 4 C-Atomen, der ein bis drei Chlor-, Brom- oder Fluoratome enthält, einen Alkylrest mit 2 bis 4 C-Atomen, der durch eine Alkoxygruppe mit 1 bis 3 C-Atomen im Alkyl, die mindestens durch zwei C-Atome von X entfernt ist, substituiert ist, einen Alkylrest mit 1 bis 3 C-Atomen, der durch eine Cycloalkylgruppe mit 3 bis 6 C-Atomen im Ring, die gegebenenfalls ein oder zwei Alkylgruppen mit 1 bis 3 C-Atomen aufweist, substituiert ist, einen Alkylrest mit 1 bis 4 C-Atomen, der durch eine Phenylgruppe, gegebenenfalls gemäß Anspruch 1 substituiert, substituiert ist, einen Cycloalkylrest mit 3 bis 6 C-Atomen im Ring, gegebenenfalls gemäß Anspruch 1 substituiert, einen Alkenylrest mit 3 bis 5 C-Atomen, einen Alkinylrest mit 3 bis 5 C-Atomen oder einen Phenylrest, gegebenenfalls gemäß Anspruch 1 substituiert, bedeuten.

3. Verbindungen der Formel I nach Anspruch 1, in denen $R^1$ Methyl und $R^2$ einen Alkylrest mit 1 bis 4 C-Atomen, der gegebenenfalls 1- bis 3fach durch Chlor, Brom oder Fluor substituiert ist, einen Cycloalkylrest mit 3 bis 6 C-Atomen im Ring und gegebenenfalls gemäß Anspruch 1 substituiert, einen Benzylrest, in dem der Phenylring gegebenenfalls gemäß Anspruch 1 substituiert ist, oder einen Alkinylrest mit 3 bis 5 C-Atomen bedeuten.

4. Verfahren zur Herstellung von Verbindungen der Formel I nach Anspruch 1, dadurch gekennzeichnet, daß man

a) ein Aminophenyl-dihydropyridazinon der Formel II,

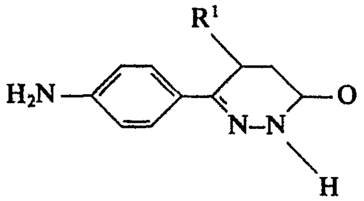

(II)

in der $R^1$ die für Formel I angegebenen Bedeutungen hat, mit einer Verbindung der Formel III,

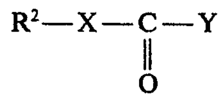

(III)

in der X und $R^2$ die für Formel I angegebenen Bedeutungen haben und für Y ein Halogenatom steht, in an sich bekannter Weise umsetzt oder,

b) daß man eine Aminosäure der Formel IV,

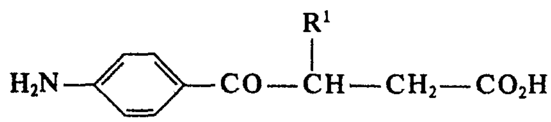

(IV)

in der $R^1$ die für Formel I angegebenen Bedeutungen hat, mit einer Verbindung der Formel III,

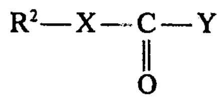

(III)

in der $R^2$, X und Y die oben angegebenen Bedeutungen haben, umsetzt und die entstandene Verbindung der Formel V

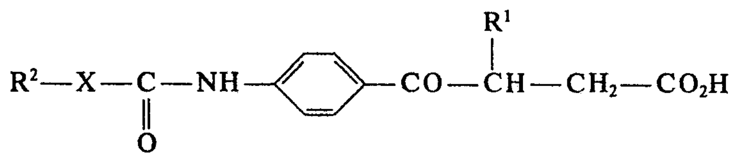

(V)

mit Hydrazin cyclisiert oder,

c) daß man ein Isocyanat der Formel VI,

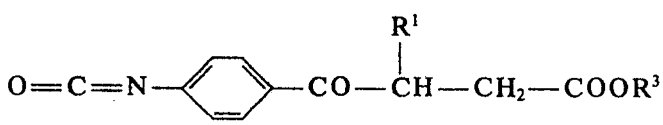

(VI)

in der $R^1$ die für Formel I angegebenen Bedeutungen hat und $R^3$ für einen Alkylrest mit 1 bis 3 C-Atomen steht, mit einer Verbindung der Formel VII,

$R^2-X-H$ (VII)

in der $R^2$ und X die für Formel I angegebenen Bedeutungen haben, umsetzt und die erhaltene Verbindung der Formel VIII

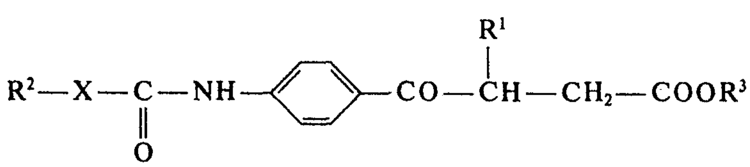

(VIII)

mit Hydrazin cyclisiert.

5. Therapeutisches Mittel, gekennzeichnet durch einen Gehalt an einer Verbindung der Formel I nach Anspruch 1 als Wirkstoff neben üblichen Trägerstoffen und Verdünnungsmitteln.

6. Eine Verbindung der Formel I nach Anspruch 1 als Arzneimittel bei der Behandlung von hohem Blutdruck oder thrombo-embolischen Erkrankungen.

**Claims**

1. A compound of the formula I

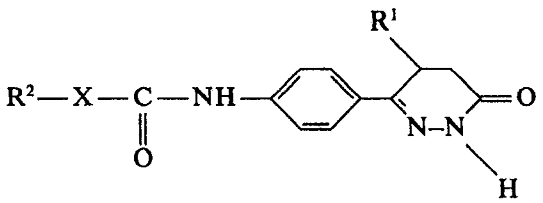

(I)

where X is oxygen or sulfur, $R^1$ is alkyl of 1 to 3 carbon atoms, and $R^2$ is alkyl of 1 to 8 carbon atoms, which is unsubstituted or is substituted by one to four halogen atoms, by an alkoxy group, where alkyl is of 1 to 4 carbon atoms, which is separated from X by not less than two carbon atoms, by a cycloalkyl group of 3 to 8 carbon atoms in the ring, which may or may not bear one or two alkyls of 1 to 3 carbon atoms, or by a phenyl radical which is unsubstituted or bears up to three identical or different substituents chosen from alkyl of 1 to 3 carbon atoms, alkoxy of 1 to 3 carbon atoms, halogen, trifluoromethyl or nitro, or is cycloalkyl of 3 to 8 carbon atoms in the ring, which is unsubstituted or substituted by up to four alkyls of 1 to 4 carbon atoms, or is alkenyl of 3 to 8 carbon atoms or alkynyl of 3 to 8 carbon atoms, or is phenyl which is unsubstituted or bears up to three identical or different substituents chosen from alkyl of 1 to 4 carbon atoms, alkoxy, where alkyl is of 1 to 4 carbon atoms, halogen, trifluoromethyl, cyano or nitro.

2. A compound of the formula I as claimed in claim 1, where $R^1$ is methyl, and $R^2$ is alkyl of 1 to 4 carbon atoms which is unsubstituted or contains up to three chlorine, bromine or fluorine atoms, alkyl of 2 to 4 carbon atoms which is substituted by alkoxy, where alkyl is of 1 to 3 carbon atoms, which is separated from X by not less than two carbon atoms, alkyl of 1 to 3 carbon atoms which is substituted by cycloalkyl which has 3 to 6 carbon atoms in the ring and may or may not bear one or two alkyls of 1 to 3 carbon atoms, alkyl of 1 to 4 carbon atoms which is substituted by phenyl which itself is unsubstituted or substituted as specified in claim 1, cycloalkyl of 3 to 6 carbon atoms in the ring, which is unsubstituted or substituted as specified in claim 1, alkenyl of 3 to 5 carbon atoms, alkynyl of 3 to 5 carbon atoms or phenyl which is unsubstituted or substituted as specified in claim 1.

3. A compound of the formula I as claimed in claim 1, where $R^1$ is methyl, and $R^2$ is alkyl of 1 to 4 carbon atoms which is unsubstituted or contains up to 3 chlorine, bromine and fluorine atoms, or is cycloalkyl which has 3 to 6 carbon atoms in the ring and is unsubstituted or substituted as specified in claim 1, or is benzyl in which the phenyl ring is unsubstituted or substituted as specified in claim 1, or is alkynyl of 3 to 5 carbon atoms.

4. A process for the preparation of a compound of the formula I as claimed in claim 1, wherein

(a) an aminophenyl-dihydropyridazinone of the formula II

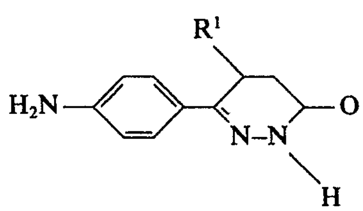

(II)

where $R^1$ has the meanings given for formula I, is reacted in a conventional manner with a compound of the formula III

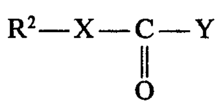

(III)

where X and $R^2$ have the meanings given for formula I, and Y is halogen, or

(b) an aminoacid of the formula IV

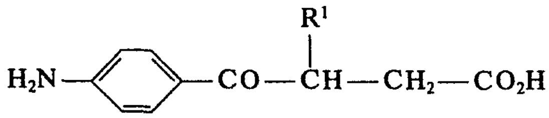

(IV)

where $R^1$ has the meanings given for formula I, is reacted with a compound of the formula III

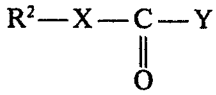

(III)

where $R^2$, X and Y have the meanings given above, and the resulting compound of the formula V

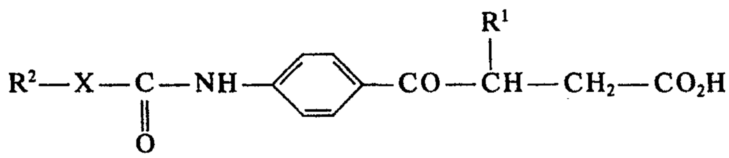

(V)

is cyclized with hydrazine, or

(c) an isocyanate of the formula VI

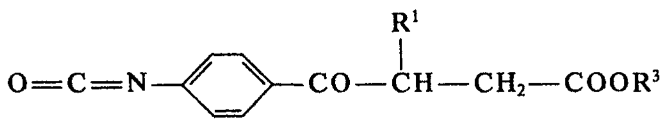

(VI)

where $R^1$ has the meanings given for formula I, and $R^3$ is alkyl of 1 to 3 carbon atoms, is reacted with a compound of the formula VII

$$R^2-X-H \qquad (VII)$$

where $R^2$ and X have the meanings given for formula I, and the resulting compound of the formula VIII

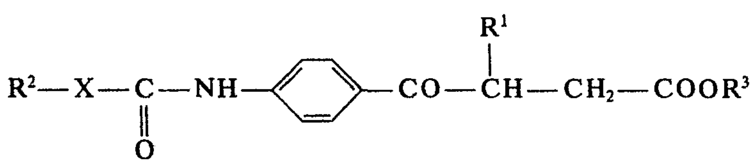

(VIII)

is cyclized with hydrazine.

5. A therapeutic agent containing a compound of the formula I as claimed in claim 1, as active ingredient, as well as conventional carriers and diluents.

6. A compound of the formula I as claimed in claim 1 as a drug for the treatment of high blood pressure and thrombo-embolic disorders.

## Revendications

1. Composés de la formule I

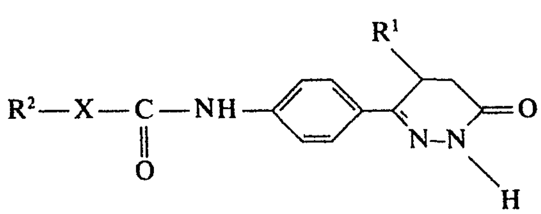

(I)

dans laquelle

X désigne un atome d'oxygène ou de soufre,
$R^1$ un radical alkyle en $C_1$ à $C_3$ et
$R^2$ un radical alkyle en $C_1$ à $C_8$, éventuellement substitué par un à quatre atomes d'halogène, par un groupe alcoxy à radical alkyle en $C_1$ à $C_4$, séparé de X par au moins deux atomes de carbone, par un groupe cycloalkyle avec un noyau cyclique avec trois à huit atomes de carbone, portant éventuellement un ou deux radicaux alkyle en $C_1$ à $C_3$, ou par un groupe phényle éventuellement mono-, di- ou tri-substitué par des substituants alkyle en $C_1$ à $C_3$, alcoxy à radical alkyle en $C_1$ à $C_3$, halogène, trifluorométhyle ou nitro identiques ou différents, un groupe cycloalkyle avec un noyau cyclique avec trois à huit atomes de carbone, portant éventuellement un à quatre substituants alkyle en $C_1$ à $C_4$, un radical alcényle en $C_3$ à $C_8$, un radical alcynyle en $C_3$ à $C_8$ ou un groupe phényle éventuellement mono- à tri-substitué par des substituants alkyle en $C_1$ à $C_4$, alcoxy à radical alkyle en $C_1$ à $C_4$, halogène, trifluorométhyle, cyano ou nitro identiques ou différents.

2. Composés de la formule I selon la revendication 1, pour lesquels $R^1$ est un radical méthyle et $R^2$ désigne un radical alkyle en $C_1$ à $C_4$, un radical alkyle en $C_1$ à $C_4$ portant un à trois atomes de chlore, de brome ou de fluor, un radical alkyle en $C_2$ à $C_4$, substitué par un groupe alcoxy à radical alkyle en $C_1$ à $C_3$, séparé de X par au moins deux atomes de carbone, un radical alkyle en $C_1$ à $C_3$, substitué par un groupe cycloalkyle avec un noyau cyclique avec trois à six atomes de carbone, portant éventuellement un ou deux radicaux alkyle en $C_1$ à $C_3$, un radical alkyle en $C_1$ à $C_4$, substitué par un groupe phényle lui-même éventuellement substitué comme indiqué dans la revendication 1, un groupe cycloalkyle avec un noyau cyclique avec trois à six atomes de carbone, éventuellement substitué comme indiqué dans la revendication 1, un radical alcényle en $C_3$ à $C_5$, un radical alcynyle en $C_3$ à $C_5$ ou un groupe phényle éventuellement substitué comme indiqué dans la revendication 1.

3. Composés de la formule I selon la revendication 1, pour lesquels $R^1$ est un radical méthyle et $R^2$ désigne un radical alkyle en $C_1$ à $C_4$, éventuellement mono-, di- ou tri-substitué par des atomes de chlore, de brome ou de fluor, un groupe cycloalkyle avec un noyau cyclique avec trois à six atomes de carbone, éventuellement substitué comme indiqué dans la revendication 1, un groupe benzyle, dont le noyau phénylique est éventuellement substitué comme indiqué dans la revendication 1, ou un groupe alcynyle en $C_3$ à $C_5$.

4. Procédé de préparation de composés de la formule I selon la revendication 1, caractérisé en ce que:

a) on fait réagir une aminophényl-dihydro-pyridazinone de la formule II

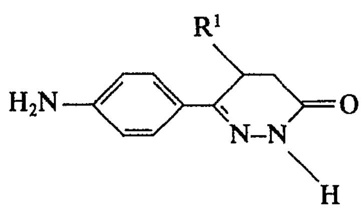

(II)

dans laquelle $R^1$ possède les significations définies pour la formule I, d'une manière connue en soi avec un composé de la formule III

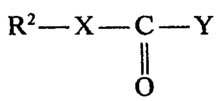

(III)

dans laquelle X et $R^2$ possèdent les significations définies pour la formule I et Y désigne un atome d'halogène; ou

b) on fait réagir un acide aminé de la formule IV

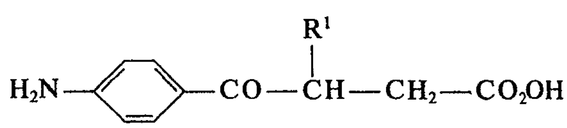

(IV)

dans laquelle $R^1$ possède les significations définies pour la formule I, avec un composé de la formule III

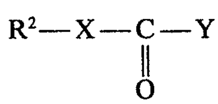

(III)

dans laquelle $R^2$, X et Y possèdent les significations définies, puis on cyclise le composé obtenu, de la formule V

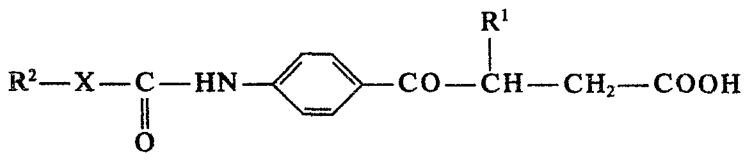

(V)

à l'aide d'hydrazine; ou

c) on fait réagir un isocyanate de la formule VI

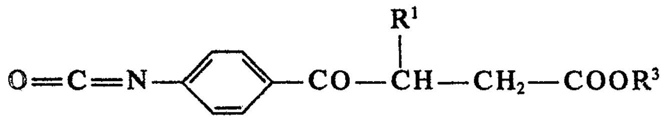

(VI)

dans laquelle $R^1$ possède les significations définies pour la formule I et $R^3$ désigne un radical alkyle en $C_1$ à $C_3$, avec un composé de la formule VII

$R^2-X-H$ (VII)

dans laquelle $R^2$ et X possèdent les significations définies pour la formule I, puis on cyclise le composé obtenu, de la formule VIII

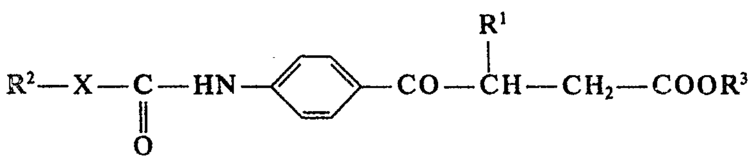

(VIII)

à l'aide d'hydrazine.

5. Composition thérapeutique, caractérisée en ce qu'elle contient, à côté de matières supports et de diluants usuels, un composé de la formule I selon la revendication 1 comme principe actif.

6. Composé de la formule I selon la revendication 1, utilisé comme substance thérapeutique pour le traitément de l'hyper-tension et d'affections thrombo-emboliques.